# EUROPEAN PATENT SPECIFICATION

(11) **EP 4 262 900 B1**
(45) Date of publication and mention of the grant of the patent: **08.07.2026**
(21) Application number: 21843769.7
(22) Date of filing: 15.12.2021
(51) Int. Cl.: A61L 9/20, C02F 1/32, F21V 7/00

(54) **DEVICE FOR DISINFECTING A FLUID FLOW IN A CONDUIT BY MEANS OF UV-C RADIATION**
VORRICHTUNG ZUR DESINFEKTION EINES FLUIDSTROMS IN EINER LEITUNG MITTELS UV-C-STRAHLUNG
DISPOSITIF DE DÉSINFECTION D'UN FLUX DE FLUIDE DANS UN CONDUIT AU MOYEN D'UN RAYONNEMENT UV-C

(30) Priority: 15.12.2020 IT 202000030899
(43) Date of publication of application: 25.10.2023
(73) Proprietor: Istituto Nazionale di Astrofisica, 00136 Roma (RM) (IT)
(72) Inventor: LOMBINI, Matteo, 00136 ROMA (IT); BIANCO, Andrea, 00136 ROMA (IT); CORTECCHIA, Fausto, 00136 ROMA (IT); DIOLAITI, Emiliano, 00136 ROMA (IT); LESSIO, Luigi, 00136 ROMA (IT); MALAGUTI, Giuseppe, 00136 ROMA (IT); PARESCHI, Giovanni, 00136 ROMA (IT); ZANUTTA, Alessio, 00136 ROMA (IT)
(74) Representative: Studio Torta S.p.A.
(86) International application number: PCT/IB2021/061779
(87) International publication number: WO 2022/130246

(56) References cited:
- US-A- 5 874 741
- US-A1- 2015 246 148
- US-A9- 2018 008 741

## Description

### TECHNICAL FIELD

The present invention relates to a device for disinfecting a fluid flow in a conduit by means of UV-C radiation. The invention is preferably applied for disinfecting air in conduits of air conditioning or ventilation systems in general, which will be hereinafter referred to without losing generality. An alternative application is for disinfecting water or other fluids.

### BACKGROUND ART

In this emergency period related to the Covid-19 pandemic, controlling the virus spreading in the air is one of the most important tools for reducing disease transmission.

It has been shown that the virus can spread through the air, typically in aerosols generated from coughing, sneezing and breathing by infected people, over long distances and for considerable periods of time.

In particular, cases of virus transmission through ventilation conduits have been proven; this constitutes a non-negligible risk factor in all public places (offices, shops, department stores, means of mass transport) or private environments subject to air recirculation.

The disinfecting power of UV radiation is well known; however, UV disinfection systems known to be effective, particularly with wavelengths operating in the so-called UV-C band (ranging from 250 to 280 nm), require a dosage of several mJ/cm² (varying depending on the micro-organism considered) that can only be achieved by using relatively high-power sources and/or a relatively long residence time of the air in a confined area subject to radiation.

It follows that the known systems have considerable application limits. For example, they are not adapted to disinfect high flow-rate flows such as those within ventilation conduits.

US 2018/008741 A9 discloses a device for disinfecting a fluid flow in accordance with the preamble of claim 1.

### DISCLOSURE OF INVENTION

Aim of the present invention is to make a device for disinfecting a fluid flow which is free from the drawbacks of the known and above-specified devices.

The aforesaid aim is achieved by a device according to claim 1.

Thanks to the curved profile of the device walls, radiation is concentrated in the device itself, thus enhancing the sterilising action. This makes it possible to sterilise larger flow rates of fluid.

### BRIEF DESCRIPTION OF THE DRAWINGS

For a better understanding of the present invention, some preferred embodiments are described below with reference to the accompanying drawings, wherein:
figure 1 is a schematic median section of a disinfection device according to a first embodiment of the invention;
figure 2 is a section of the device along line II-II in figure 1;
figure 3 is a schematic section of a second embodiment of the invention; and
figure 4 is a section of a device according to the comparative examples.

### BEST MODE FOR CARRYING OUT THE INVENTION

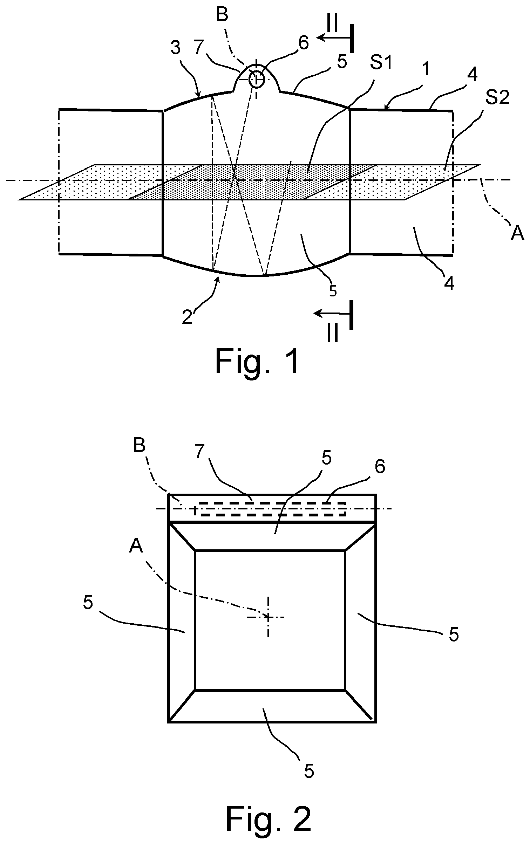

With reference to figure 1, number 1 denotes a ventilation conduit of axis A comprising a disinfecting device 2 according to the present invention.

The conduit 1 has a constant cross-section throughout its length, e.g. 300 × 300 mm square, apart from an intermediate tract 3 constituting a casing for the disinfection device 2.

The casing 3 has walls 5 with a curved profile, connected with the plane walls 4 of the conduit 1 to the respective axial ends; in particular, each wall 5, seen in section with a longitudinal plane perpendicular to the walls 5 connected thereto, has a curved profile with the concavity facing the interior of the casing 3.

The casing 3 therefore has a "convex" shape with a square cross-section varying from a minimum section at the ends, coinciding with the section of the conduit 1, to a maximum section at a longitudinal centreline of the casing.

In the example shown, the device 2 has an axial length of 0.5 m, and the radius of curvature of the walls 5 with a curved profile is constant and equal to 1 m; the walls 5 are therefore made up of portions of a cylinder with an axis perpendicular to the axis A.

The conduit 1 (partially shown) has an overall length of 5.5 m (including the device 2).

The device 2 comprises a source 6 of UV-C radiation which, in the example shown, consists of a mercury vapour discharge lamp.

The source 6 has an elongated cylindrical shape, of axis B, in the example shown having a length of 240 mm and a radius of 9 mm.

### Example 1 (figures 1 and 2)

The source 6 is arranged with the axis B transverse to the conduit 1 inside a parabolic reflector 7 defining a transverse recess along a centreline of a wall 4.

The reflector 7 has a maximum width of 100 mm and a depth of 50 mm, with a minimum radius of curvature of 25 mm at the apex of the parabola. The section of maximum amplitude, wherein the reflector joins the wall 5, is placed at 180 mm from the axis A.

The axis of the source 6 is located in the focus of the reflector 7 at a distance of approximately 395 mm from the opposite wall 5.

The inner surfaces of the reflector 7, walls 4 and walls 5 are reflective.

In this disclosure and in the claims, the term "reflective" means a surface having a reflectivity greater than 80%.

In this specific example, the reflectivity considered is equal to 93%. An example of a usable material is Miro^{®} UVC from Alanod GmbH & Co. KG.

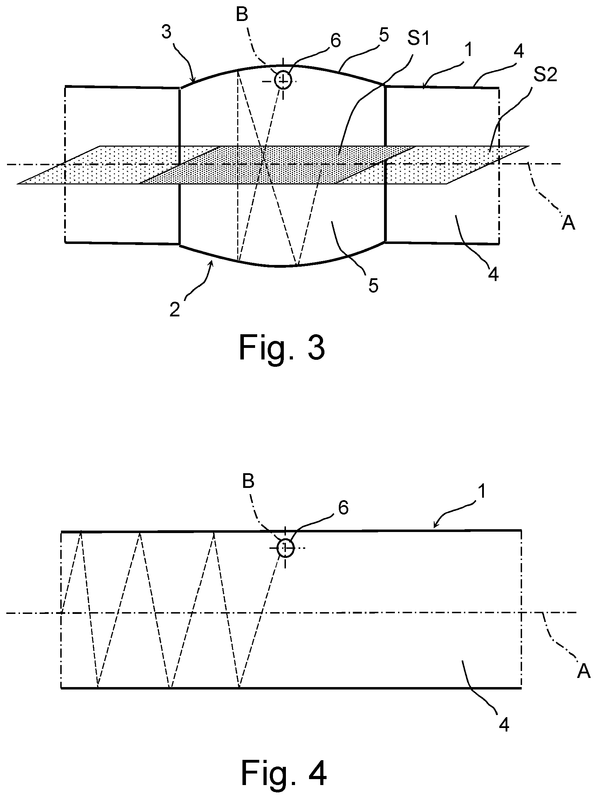

### Example 2 (figure 3)

The example 2 differs from the example 1 in that the source 6 is arranged inside the casing 3, along a centreline of a wall 5 of the device 2.

### Comparative example 1 (figure 4)

The comparative example 1 differs from examples 1 and 2 in that the walls 5 of the device 2 are not curved. Therefore, the conduit 1 has a constant square cross-section.

The source 6 is arranged with the axis B transverse with respect to the conduit 1 in proximity of a wall 4 of conduit 1.

The reflectivity of the inner walls of the conduit 1 is as in example 1.

### Comparative example 2 (figure 4)

The comparative example 2 is the same as comparative example 1, but the reflectivity of the inner surfaces of conduit 1 is low (5%).

### Comparison

In order to evaluate the performance of the disinfection device 2 according to the various examples, the irradiation was calculated in two detection rectangular longitudinal median sections S1 and S2 (figures 1, 3), placed along the axis A and parallel to the axis B of the source 6, having a width equal to the width of the conduit 1 (300 mm) and extending in length respectively for the length of the single device 2 (0.5 m) and for the entire length of the conduit 1 (5.5 m).

For ease of comparison, a unit power (1 W) of the source was considered.

For simplicity, it was assumed that the flow in the conduit is laminar with a constant velocity, and that the velocity remains unchanged inside the device 2 despite the increase in area. This is a basically plausible and certainly conservative hypothesis.

The total radiation power on sections S1 and S2 was then detected for each example.

Finally, the average power on sections S1 and S2 was calculated for each example by dividing the total power by the area of the respective sections.

The results are summarised in the table below:

| | **Example 1** | **Example 2** | **Comparative examples** | |
|---|---|---|---|---|
| | | | **1** | **2** |
| **Total power S1** | 4990 *mW* | 2860 *mW* | 1410 *mW* | 225 *mW* |
| **Total power S2** | 5030 *mW* | 3090 *mW* | 1700 *mW* | 271 *mW* |
| **Power density S1 (average)** | 3.3 *mW* / *cm*² | *1.9 mW* / *cm*² | 0.94 *mW* / *cm²* | 0.15 *mW* / *cm*² |
| **Power density S2 (average)** | 0.30 *mW* / *cm*² | 0.18 *mW* / *cm*² | 0.1 *mW* / *cm*² | 0.016 *mW* / *cm*² |

From an examination of the results, it is clear that the use of a device 2 with curved profile side walls (example 2) leads substantially to a doubling of the average power density inside the device, due essentially to the greater permanence of the light beams generated by the source 6 inside the device 2, compared to a conduit with a constant cross-section (comparative example 1). It must be noted in particular the path of a radiation beam in figure 3, wherein the multiple reflections on the walls 5 tend to keep the beam inside the device 2, compared to the beam in figure 4 which tends to disperse along the conduit 1.

It is worth noting that in the comparative example 1, the total power in the detection section S2 exceeds the one in section S1 by approximately 20% (i.e., more than 20% of the power emitted by the source 6 "exits" the device 2 and is dispersed in the rest of the conduit), whereas in example 2, the total power in the detection section S2 exceeds the one in section S1 by approximately 8%, denoting a greater power concentration in the device 2.

By comparing the comparative example 1 and comparative example 2, both of which are not part of the present invention, it is possible to evaluate the contribution of reflectivity, with the geometrical characteristics of the conduit 1 and the source 6 being equal.

Comparing example 1 with example 2, it can be seen that the arrangement of the source 2 outside the curved profile of the wall 4 and inside the reflector 7 allows the power in the detection section S1 to be increased by about 75%, and the difference between the total power on section S2 and that on section S1 to be reduced to less than 1%. With this solution, therefore, the power emitted by the source 6 is almost entirely concentrated in device 2.

In order to calculate the radiation [mJ/cm²], it is sufficient to multiply the above-set forth average power values by the residence time of the flow inside the device 2 and conduit 1 respectively (and by the power of the source 6, which has so far been considered as unitary).

Finally, it is clear that modifications and variations can be made to the described embodiments without departing from the scope of protection defined by the claims.

In particular, the conduit 1 and the device 2 may be circular rather than square in cross-section; thus, the device 1 may be delimited by a continuous toroidal wall rather than walls curved in one direction only.

The profile of the side wall(s) of the device 2, in longitudinal section, may be defined by a curve with variable, rather than constant, radius, for example a parabola.

The source 6 can be of different types and operate at different wavelengths as long as they are effective in sanitising micro-organisms. For example, it may consist of an excimer lamp (operating at 222 nm) or one or more substantially point sources, such as LEDs (operating at approximately 275 nm), or a curved profile tubular source.

There could also be more sources in case more irradiation is needed. The sources could each be in their own reflector or arranged in the same reflector

The device 2 may be used to sterilise water (or another liquid) in a conduit. In this case, the above-noted advantages are joined by the further advantage that a liquid slows down in the device 2, due to the convex shape of the walls 5, and thus increases the residence time within the device itself.

## Claims

1. A device for disinfecting a fluid in a conduit (1) comprising:
- an outer casing (3) adapted to be connected to at least a portion of said conduit (1) and having a longitudinal axis (A); and
- at least one source (6) of UV-C radiation housed in the casing (3),
the casing (5) having a reflective inner surface and including at least one side wall (5) having a longitudinal section with a curved profile with the concavity facing the interior of the casing (5),
**characterized in that** the casing (3) has a square cross section and comprises four walls (5) having said curved profile in longitudinal section.

2. A device as claimed in claim 1, wherein the source (6) is arranged in proximity to the at least one side wall (5) of the casing (3).

3. A device as claimed in claim 1 or 2, wherein the source (6) is disposed within a reflector (7) defining a cavity open towards the inside of the casing (3).

4. A device as claimed in claim 3, wherein the reflector (7) defines a recess of said at least one side wall (5).

5. A device as claimed in any of the preceding claims, wherein the source (6) is a mercury vapor discharge lamp.

6. A device as claimed in any of claims 1 to 4, wherein the source (6) is an excimer lamp or includes one or more LEDs.

7. A device as claimed in any of the preceding claims, wherein the source (6) is an elongated cylindrical lamp having a longitudinal axis (B) perpendicular to the axis (A) of the housing (3).

## Patentansprüche

1. Vorrichtung zur Desinfektion eines Fluids in einer Leitung (1), umfassend:
- ein äußeres Gehäuse (3), das dazu geeignet ist, mit mindestens einem Abschnitt der Leitung (1) verbunden zu werden, und eine Längsachse (A) aufweist, und
- mindestens eine UV-C-Strahlungsquelle (6), die in dem Gehäuse (3) untergebracht ist,
wobei das Gehäuse (5) eine reflektierende innere Oberfläche aufweist und mindestens eine Seitenwand (5) beinhaltet, die einen Längsschnitt mit einem gekrümmten Profil aufweist, wobei der Hohlraum dem Inneren des Gehäuses (5) zugewandt ist,
**dadurch gekennzeichnet, dass** das Gehäuse (3) einen quadratischen Querschnitt aufweist und vier Wände (5) umfasst, die das gekrümmte Profil im Längsschnitt aufweisen.

2. Vorrichtung nach Anspruch 1, wobei die Quelle (6) in der Nähe der mindestens einen Seitenwand (5) des Gehäuses (3) angeordnet ist.

3. Vorrichtung nach Anspruch 1 oder 2, wobei die Quelle (6) innerhalb eines Reflektors (7) angeordnet ist, definierend einen Hohlraum, der in Richtung der Innenseite des Gehäuses (3) offen ist.

4. Vorrichtung nach Anspruch 3, wobei der Reflektor (7) eine Ausnehmung der mindestens einen Seitenwand (5) definiert.

5. Vorrichtung nach einem der vorhergehenden Ansprüche, wobei die Quelle (6) eine Quecksilberdampfentladungslampe ist.

6. Vorrichtung nach einem der Ansprüche 1 bis 4, wobei die Quelle (6) eine Excimerlampe ist oder eine oder mehrere LEDs beinhaltet.

7. Vorrichtung nach einem der vorhergehenden Ansprüche, wobei die Quelle (6) eine langgestreckte zylindrische Lampe, aufweisend eine Längsachse (B) senkrecht zu der Achse (A) des Gehäuses (3), ist.

## Revendications

1. Dispositif de désinfection d'un fluide dans un conduit (1) comprenant :
- une enveloppe extérieure (3) adaptée pour être raccordée à au moins une partie dudit conduit (1) et présentant un axe longitudinal (A) ; et
- au moins une source (6) de rayonnement UV-C logée dans l'enveloppe (3), l'enveloppe (5) comportant une surface intérieure réfléchissante et
comprenant au moins une paroi latérale (5) présentant une section longitudinale avec un profil incurvé, la concavité étant orientée vers l'intérieur de l'enveloppe (5),
**caractérisé en ce que** l'enveloppe (3) présente une section transversale carrée et comprend quatre parois (5) ayant ledit profil incurvé en section longitudinale.

2. Dispositif selon la revendication 1, la source (6) étant disposée à proximité d'au moins une paroi latérale (5) de l'enveloppe (3).

3. Dispositif selon la revendication 1 ou 2, la source (6) étant disposée à l'intérieur d'un réflecteur (7) définissant une cavité ouverte vers l'intérieur de l'enveloppe (3).

4. Dispositif selon la revendication 3, le réflecteur (7) définissant une cavité de ladite au moins une paroi latérale (5).

5. Dispositif selon l'une quelconque des revendications précédentes, la source (6) étant une lampe à décharge à vapeur de mercure.

6. Dispositif selon l'une quelconque des revendications 1 à 4, la source (6) étant une lampe à excimère ou comprenant une ou plusieurs DEL.

7. Dispositif selon l'une quelconque des revendications précédentes, la source (6) étant une lampe cylindrique allongée dont l'axe longitudinal (B) est perpendiculaire à l'axe (A) du logement (3) .
